# EUROPEAN PATENT SPECIFICATION

(11) **EP 0 799 421 B1**
(45) Date of publication and mention of the grant of the patent: **19.02.2003**
(21) Application number: 95943861.5
(22) Date of filing: 18.12.1995
(51) Int. Cl.: G01N 33/534, G01N 33/58, G01N 33/569, G01N 33/571, C12Q 1/68

(54) **DIFFERENTIAL TIMING METHOD FOR DETECTING MULTIPLE ANALYTES IN A TEST SAMPLE**
DIFFERENZIERTES TIMINGSVERFAHREN ZUM NACHWEISS MULTIPLE ANALYTE IN EINE TESTPROBE
PROCEDE DE DETECTION, PAR MESURE DE TEMPS DIFFERENTIELLE, DE PLUSIEURS ANALYTES DANS UN ECHANTILLON TEST

(30) Priority: 22.12.1994 US 362036
(43) Date of publication of application: 08.10.1997
(73) Proprietor: Abbott Laboratories, Abbott Park, Illinois 60064-3500 (US)
(72) Inventor: KHALIL, Omar, S., Libertyville, IL 60048 (US); HANLEY, Kathleen, A., Grayslake, IL 60030 (US)
(74) Representative: Modiano, Guido, Dr.-Ing.
(86) International application number: US9516464
(87) International publication number: WO96019731

(56) References cited:
- EP-A- 0 238 353
- WO-A-92/12255
- WO-A-94/18570
- CLINICAL CHEMISTRY, vol. 32, no. 9, 1986, WINSTON-SALEM NC USA, pages 1682-1686, XP000567059 J. KANG ET AL: "A high sensitive immunoenzymometric assay involving "common-capture" partcles and membrane filters."

### SEQUENCE LISTING

(1) GENERAL INFORMATION:
   (i) APPLICANT: Omar S. Khalil
      Kathleen A. Hanley
   (ii) TITLE OF INVENTION: DIFFERENTIAL TIMING METHOD FOR DETECTING MULTIPLE AMALYTES IN A TEST SAMPLE
   (iii) NUMBER OF SEQUENCES: 3
   (iv) CORRESPONDENCE ADDRESS:
      (A) ADDRESSEE: Abbott Laboratories
      (B) STREET: 100 Abbott Park Road
      (C) CITY: Abbott Park
      (D) STATE: Illinois
      (E) COUNTRY: USA
      (F) ZIP: 60064-3500
   (v) COMPUTER READABLE FORM:
      (A) MEDIUM TYPE: Floppy disk
      (B) COMPUTER: Macintosh
      (C) OPERATING System: 7.0.1
      (D) SOFTWARE: MS Word
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Paul D. Yasger
      (B) REGISTRATION NUMBER: 37,477
      (C) DOCKET NUMBER: 5365.US.01
   (ix) TELECOMMUNICATION INFORMATION:
      (A) TELEPHONE: 708/937-2341
      (B) TELEFAX: 708/938-2623
      (C) TELEX:
(2) INFORMATION FOR SEQ ID NO:1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: synthetic DNA
   (ix) FEATURE:
      (A) NAME/KEY: 5' carbazole
      (B) LOCATION: 1
   (ix) FEATURE:
      (A) NAME/KEY: 3' adamantane
      (B) LOCATION: 30
   (xi) SEQUENCE DESCRIPTION: SEQ ID NO:1:
   TTGTTTGAAC CGCATCTGAG CTTTTTTATC 30
(2) INFORMATION FOR SEQ ID NO:2:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: synthetic DNA
   (ix) FEATURE:
      (A) NAME/KEY: 5' carbazole
      (B) LOCATION: 1
   (ix) FEATURE:
      (A) NAME/KEY: 5' adamantane
      (B) LOCATION: 48
   (xi) SEQUENCE DESCRIPTION: SEQ ID NO:2:
   GACTTTGCAA CTCTTGGTGG TAGACTTGGT CATAATGGAC TTTTGTTG 48
(2) INFORMATION FOR SEQ ID NO:3:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: synthetic DNA
   (ix) FEATURE:
      (A) NAME/KEY: 5' fluorescein
      (B) LOCATION: 1
   (ix) FEATURE:
      (A) NAME/KEY: 3' biotin
      (B) LOCATION: 48
   (xi) SEQUENCE DESCRIPTION: SEQ ID NO:3:
   GGCAAGGCCA ATGGACATAT CAAATTTATC AAGAGCCATT TAAAAATC 48

## Description

### Field of the Invention

The present invention relates to immunoassay methods, and, in particular, relates to an immunoassay method for detecting the presence or amount of multiple analytes that may be present in a test sample.

### Background of the Invention

Techniques for performing immunoassays that detect the presence or amount of an analyte in a test sample are generally well known in the art. For example, conventional enzyme immunoassay procedures involve a series of steps wherein an analyte in a test sample is initially bound to a corresponding antigen or antibody reagent. A second antigen or antibody which has been labeled or conjugated with an enzyme or other substance capable of producing a detectable signal is then introduced into the sample and a signal can then be read to indicate the presence or amount of the antigen or antibody in the test sample.

Solid phase immunoassay procedures are often times preferred over other diagnostic methods because of their safety, ease of use, specificity and sensitivity. Moreover when employing a solid-phase immunoassay procedure, the result is easily observed by instruments which further increase the accuracy of the procedure.

One form of a conventional solid-phase immunoassay is a "sandwich assay" which involves contacting a test sample suspected of containing an analyte with a substantially solid inert plastic, latex or glass bead or microparticle, or other support material which has been coated with a protein or another substance capable of binding the analyte to the surface of the support. The analyte and the protein or substance capable of binding the analyte are commonly referred to as a "specific binding pair" or, individually, known as "specific binding members". After the analyte is bound to the support material the remaining test sample is removed from the support and the analyte bound support material is treated with a second binding member. The second binding member can be conjugated with a detectable moiety such as an enzyme, a fluorophore or a chemiluminescent label and collectively, the binding member/detectable moiety complex is variably referred to as a conjugate. The conjugate becomes bound to the analyte which is bound on the support and the solid support, having the first binding member, the analyte and conjugate bound thereon is separated from any unbound conjugate, typically with one or more wash steps. In the case of an enzyme immunoassay, an indicator substance, for example, a chromogenic substrate, is added which reacts with the enzyme to produce a color change. The color change can be observed visually, or more preferably by an instrument, to indicate the presence or amount of an analyte in a test sample. For solid phase fluorescence or chemiluminescence immunoassays, fluorescent labeled binding members can be monitored using excitation at an appropriate wavelength, while chemiluminescent labeled binding members can be monitored after a reaction which chemically activates the chemiluminescent label and generates light which can be detected by photometric means. The detected light, if any, can then be used as an indication of the presence or amount of an analyte in a test sample.

The aforementioned immunoassays have served and continue to serve as effective techniques for detecting the presence or amount of an analyte in a test sample.

More recently, immunoassay techniques have been employed to detect the presence or amount of a select form of analyte in a test sample. Specifically, these techniques can be utilized to detect the products resulting from nucleic acid amplification techniques. Amplification techniques such as the ligase chain reaction (LCR) which is described in European Patent Applications EP-A-320-308, the gap ligase chain reaction (GLCR) which is described in EP-A-439-182, and the polymerase chain reaction (PCR) which is described in U.S. Patents Numbered 4,683,202 and 4,683,195 are by now generally well known in the art. These amplification processes are becoming useful clinical diagnostic tools to, for example, construct assays which detect infectious organisms in a test sample. Additionally, amplification assays have been used in research and development fields as well as forensic fields to, for example, detect genetic defects.

Nucleic acid amplification techniques typically employ "primers" or "probes" to repeatedly generate copies of a target nucleic acid sequence which is usually a small region of a much larger nucleic acid sequence. Primers and probes are themselves nucleic acid sequences that are complementary to regions of a target sequence and under suitable conditions, hybridize or bind to the complementary portions of the target sequence. Copies of the target sequence are typically generated by the process of primer or probe extension which utilizes enzymes such as polymerase or ligase, separately or in combination, to add nucleotides to the hybridized primers or probes. The nucleotides that are added to the primers or probes are also complementary to the target sequence. Once the primers or probes have been sufficiently extended and/or ligated they are separated from the target sequence and a sequence complementary to the target sequence is formed. A new round of extension can then take place to amplify the number of complementary target sequences. Additionally, the sequences that are complementary to the target sequence can serve as templates for primer or probe extension to thereby amplify the number of target sequences. Hence multiple copies of the target sequence and its complementary sequence are produced.

The presence or amount of amplified target sequences and/or sequences complementary to the target sequence in a solution can be detected in much the same manner as more traditional analytes. For example, a sandwich assay format such as that previously discussed can be employed to detect the presence or amount of amplified nucleic acid sequences in a solution. Specifically, the sequences can be immobilized to a capture reagent such as, for example, a suspension of microparticles coated with a specific binding member, by incorporating a specific binding member, which forms a specific binding pair with the capture reagent's specific binding member, into the amplified sequences. Accordingly, when the sequences are contacted with the capture reagent, the sequences become immobilized to the capture reagent. Alternatively, the sequences can be immobilized to the capture reagent by contacting the sequences with a capture reagent comprising a specific binding member which directly forms a specific binding pair with the amplified sequences.

The presence or amount of the sequences immobilized to the capture reagent can be detected by directly incorporating individual nucleotides that have been labeled with detectable moieties into the amplified sequences. Alternatively, a conjugate which forms a specific binding pair with the sequences can be employed. In the case where a conjugate is employed, the conjugate can, for example, comprise a nucleic acid sequence and a fluorophore which directly binds the sequences through hydrogen bonding; or the conjugate can, for example, comprise a specific binding member and a fluorophore which covalently or otherwise binds the sequences via a specific binding member, which forms a specific binding pair with the conjugate that has been incorporated into the sequences. Once a detectable moiety has been associated with the immobilized sequences, their presence or amount, if any, can then be detected by detecting the presence of the detectable moiety.

Although the utility of immunoassay techniques as diagnostic tools is without question, it is unfortunate that clinical immunoassay techniques are generally limited to detecting the presence or amount of a single analyte in a test sample because the detection of multiple analytes requires the use of expensive and sophisticated equipment which requires significant amounts of labor and expertise in order to operate. Generally, specialized equipment is necessary because the methods currently available for detecting multiple analytes in a test sample require that the analytes are spatially segregated into areas or zones of a single analyze. Additionally, individual analytes within these zones are typically labeled with fluorescent detectable moieties which have multiple excitation and emission wavelengths. Thus, equipment that can (i) generate multiple excitation wavelengths and (ii) detect multiple emission wavelengths is necessary.

For example, European Patent Application No. EP-0-364-255 describes the simultaneous amplification of multiple nucleic acid sequences. The presence of the amplified sequences is detected using a DNA sequencing apparatus. Such a detection procedure requires sophisticated equipment which, in turn, demands significant amounts of technician interaction and time, and a correlative amount of technician training in order to use the equipment and interpret the results generated by such equipment. Accordingly, such a detection procedure is expensive and not readily amenable to automation, and therefore, not amenable to routine laboratory use.

In addition, International Patent Application No. WO 93/20227 discloses a method of amplifying multiple nucleic acid sequences in a test sample whereby the amplification products are wicked along a strip of immunochromato graphic material having an array of capture spots which are used to immobilize multiple nucleic acid sequences. The capture spots are diagonally situated across the strip of immunochromatographic material and are tantamount to a plurality of distinct capture reagents which segregate the sequences into common analyte zones which are spatially separated. The reaction products each comprise a distinct hapten which is used to segregate the products on the immunochromatographic material and a common hapten which is used for labeling the products with a selenium colloid conjugate. If reaction products are bound at the various analyte zones, visible spots are created at these zones when the selenium colloid conjugate binds the common hapten.

Similarly, Fortina et al., Molecular and Cell Probes, 6, 353-356 (1992) disclose a fluorescence based detection of a plurality of amplified DNA sequences which employs gel electrophoresis to separate the amplified sequences, and an imaging detector to detect the presence of the amplified sequences which are individually labeled with fluorophores that emit signals of varying wavelengths. According to the method disclosed therein, the labeled sequences are applied to a single lane of an agarose gel and separated into bands. Following electrophoresis, the bands are visualized via excitation of the fluorescent labels. The combination of gel electrophoresis and multicolor fluorescence also demands significant amounts of technician training and time because of the equipment necessary to run this procedure. Moreover the equipment involved, typically a gel electrophoresis apparatus and a scanning or imaging detector, make this procedure difficult to automate. WO 92/12255 describes assays for the determination of multiple analytes which may be present in a test sample by using distinct chemiluminescent labels.
WO 94/18570 relates to a one pot assay for conducting two assays for related serological disease markers in a single vessel and balancing the respective sizes of two signals to generate a combined signal. The revealing agents therein bear the same label.

### Summary of the Invention

The present invention provides a method for detecting the presence or amount of multiple analytes that may be present in a test sample without the need for segregating analytes into single analyte zones. Moreover, as taught herein, detectable moieties that have a common emission wavelength can be employed. Accordingly, the method is readily adaptable to automated operation, and is therefore amenable to routine laboratory use.

According to one embodiment, the method comprises the steps of: (a) contacting a test sample suspected of containing multiple analytes with a common capture reagent for a time and under conditions sufficient to form capture reagent/analyte complexes; (b) contacting the so-formed capture reagent/analyte complexes with at least two indicator reagents for a time and under conditions sufficient to form capture reagent/analyte/indicator reagent complexes wherein at least one of said at least two indicator reagents comprises an enzymatic label and at least one of said at least two indicator reagents comprises a fluorophore; and (c) detecting at least two measurable signals as a measure of the presence or amount of the analytes in the test sample. The signals which are measured can be differentiated based upon (i) the influence one of the signals has upon another signal, or (ii) the time at which the signals are generated and the influence one of the signals has upon another signal. It is contemplated and within the scope of the invention that step (a) and step (b) can be performed simultaneously.

A common capture reagent may include one or more specific binding members that immobilize at least two analytes that may be present in a test sample. Indicator reagents may comprise detectable moieties from at least two distinct detectable moiety classes which are attached to specific binding members. Preferably, the detectable moieties employed generate fluorescent signals which have common wavelengths.

In cases where an analyte comprises a detectable moiety of one class, a single indicator reagent comprising a detectable moiety from a different class can be employed to detect multiple analytes in a test sample. Such a case may arise when, for example, nucleic acid analytes are detected and one of the analytes is covalently bound or labeled with a detectable moiety. Nucleic acid sequences can be labeled with a detectable moiety through, for example, nick translation whereby labeled nucleotides are incorporated into a target sequence or the use of labeled PCR or LCR primers or probes. In such cases, a label bound to a nucleic acid serves as one class of detectable moiety that can be detected as taught herein.

Hence, according to another embodiment, a method for determining the presence or amount of at least two nucleic acid sequences that may be present in a test sample is provided. Typically, the nucleic acid sequences will comprise a capture region and a reporter region. According to this embodiment, the method comprises the steps of: (a) contacting a test sample suspected of containing multiple nucleic acid sequences with a common capture reagent for a time and under conditions sufficient to form capture reagent/analyte complexes; (b) contacting the so-formed capture reagent/analyte complexes with at least one indicator reagent, comprising the other one of an enzymatic label or a fluorophore, for a time and under conditions sufficient to form capture reagent/analyte/indicator reagent complexes; and (c) detecting at least two measurable signals as a measure of the presence or amount of the sequences in the test sample. The signals which are measured can be detected simultaneously, or simultaneously and sequentially. It is contemplated and within the scope of the invention that step (a) and step (b) can be performed simultaneously.

Similarly to above, a common capture reagent may include one or more specific binding members that immobilize at least two analytes that may be present in a test sample and an indicator reagent typically comprises a specific binding member bound to a detectable moiety. Preferably, the detectable moieties employed generate fluorescent signals which have common wavelengths.

### Brief Description of the Drawings

Figure 1 graphically illustrates the fluorescence profile from the sequential detection of two analytes according to the instant invention using a time dependent detectable moiety and a steady state detectable moiety.
Figure 2 graphically illustrates the fluorescence profile from the simultaneous detection of two analytes according to the instant invention using a time dependent detectable moiety and a steady state detectable moiety.
Figure 3 graphically illustrates the fluorescence profile from the sequential detection of three analytes according to the instant invention using two time dependent detectable moieties and a steady state detectable moiety.
Figure 4 graphically illustrates the fluorescence profile from the simultaneous and sequential detection of three analytes according to the instant invention using two time dependent detectable moieties and a steady state detectable moiety.

### Detailed Description of the Invention

The following definitions are applicable to the invention.

The term "analyte", as used herein, refers to the compound or composition to be detected or measured and which has at least one specific binding site such as an epitope or specific peptide or nucleic acid sequence. The analyte can be any substance for which there exists a naturally occurring binding member or for which a binding member can be prepared. Analytes include, but are not intended to be limited to, toxins, organic compounds, proteins, peptides, microorganisms, amino acids, carbohydrates, nucleic acid sequences, hormones, steroids, vitamins, drugs (including those administered for therapeutic purposes as well as those administered for illicit purposes), virus particles and metabolites of or antibodies to any of the above substances. For example, such analytes include, but are not intended to be limited to, RNA; DNA; ferritin; creatinine kinase (CK-MB); digoxin; phenytoin; phenobarbitol; carbamazepine; vancomycin; gentamycin; theophylline; valproic acid; quinidine; Ieutinizing hormone (LH); follicle stimulating hormone (FSH); estradiol; progesterone; IgE antibodies; vitamin B2 micro-globulin; glycated hemoglobin (Gly Hb); cortisol; digitoxin; N-acetylprocainamide (NAPA); procainamide; antibodies to rubella, such as rubella-IgG and rubella-IgM; antibodies to toxoplasmosis, such as toxoplasmosis IgG (Toxo-IgG) and toxoplasmosis IgM (Toxo-IgM); testosterone; salicylates; acetaminophen; hepatitis B virus surface antigen (HBsAg); antibodies to hepatitis B core antigen, such as anti hepatitis B core antigen IgG and IgM (Anti-HBC); human immune deficiency virus 1 and 2 (HTLV); hepatitis B e antigen (HBeAg); antibodies to hepatitis B e antigen (Anti-HBe); thyroid stimulating hormone (TSH); thyroxine (T4); total triiodothyronine (Total T3); free triiodothyronine (Free T3); carcinoembryoic antigen (CEA); alpha fetal protein (AFP); and drugs of abuse and controlled substances, including but not intended to be limited to, amphetamine; methamphetamine; barbiturates such as amobarbital, secobarbital, pentobarbital, phenobarbital, and barbital; benzodiazepines such as librium and valium; cannabinoids such as hashish and marijuana; cocaine; fentanyl; LSD; methapualone; opiates such as heroin, morphine, codeine, hydromorphone, hydrocodone, methadone, oxycodone, oxymorphone and opium; phencyclidine; propoxyhene; and the like. The term "analyte" also includes any antigenic substances, haptens, antibodies, macromolecules and combinations thereof. A preferred analyte is the product or products of a nucleic acid amplification reaction, variably known as an "amplicon", which are labeled with haptens recognizable by specific binding members. Analytes may also include positive control reagents.

Multiple analytes whose presence or amount in a test sample is preferably detected as taught herein are amplicons made from the DNA or RNA or HIV-1 and HIV-2; Human Papiloma Virus (HPV) variants such as, for example, HPV 16, HPV 18 and HPV 33 amplicons; *Chlamydia trachomatis* and *Niesseria gonorrhea* amplicons; as well as combinations of more traditional analytes such as FSH and LH; CEA and AFP; and combinations of thyroidal hormones.

"Specific binding member", as used herein, means a member of a binding pair, i.e., two different molecules where one of the molecules through chemical or physical means specifically binds to the other molecule. In addition to antigen and antibody specific binding pairs, other specific binding pairs include, but are not intended to be limited to, avidin and biotin, carbohydrates and lectins, complementary nucleotide sequences, effector and receptor molecules, an enzyme cofactor or substrate and an enzyme, a peptide sequence and an antibody specific for the sequence or an entire protein, dyes and protein binders, peptides and specific protein binders (e. g., ribonuclease, S-peptide and ribonuclease S-protein), and the like. Furthermore, binding pairs can include members that are analogs of the original binding member, for example, an analyze-analog or a binding member made by recombinant techniques or molecular engineering. If the binding member is an immunoreactant it can be, for example, a monoclonal or polyclonal antibody, a recombinant protein or recombinant antibody, a chimeric antibody, a mixture(s) or fragment(s) of the foregoing, as well as a preparation of such antibodies, peptides and nucleotides for which suitability for use as binding members is well known to those skilled in the art.

The term "capture reagent", as used herein, means a solid phase to which a binding member has been immobilized. Those skilled in the art will recognize that a binding member can be immobilized to a solid phase through numerous known methods including, for example, any chemical means and/or physical means that does not destroy the specific binding properties of the specific binding member.

The term "detectable moiety", as used herein, refers to any compound or conventional detectable chemical group having a detectable and measurable physical or chemical property variably referred to as a signal. Such detectable chemical group can be, but is not intended to be limited to, enzymatically active groups such as enzymes and enzyme substrates, prosthetic groups or coenzymes; spin labels; fluorescent molecules such as fluorescers and fluorogens; chromophores and chromogens; phosphorescent molecules; specifically bindable ligands such as biotin and avidin; electroactive species; radioisotopes; toxins; drugs; haptens; polysaccharides; polypeptides; liposomes; colored or fluorescent particles; colored or fluorescent microparticles and the like. Additionally, a detectable moiety can comprise a plurality of the above groups, such as for example a plurality of fluorophores immobilized to a polymer such as that described in co-owned U.S. Patent 566 1040 filed July 13, 1993 The detectable physical or chemical property associated with a detectable moiety can be detected visually or by an external means. Detectable moieties can also be classified which will be discussed below.

An "indicator reagent" or "conjugate", as used herein, may comprise a detectable moiety (label) which is coupled (attached) to a specific binding member. Coupling chemistries for synthesizing an indicator reagent are also well known in the art and can include, for example, any chemical means and/or physical means that does not destroy the specific binding properties of the specific binding member.

A "solid phase", as used herein, refers to any material which is insoluble, or can be made insoluble by a subsequent reaction. The solid phase can be chosen for its intrinsic ability to attract and immobilize a binding member to form a capture reagent Alternatively, the solid phase can retain an additional receptor which has the ability to attract and immobilize a binding member to form a capture reagent The additional receptor can include a charged substance that is oppositely charged with respect to a binding member or to a charged substance conjugated to a binding member. As yet another alternative, the receptor molecule can be any specific binding member which is immobilized upon (attached to) the solid phase and which has the ability to immobilize another binding member through a specific binding reaction. The receptor molecule enables the indirect binding of a binding member to a solid phase material before the performance of the assay or during the performance of the assay. The solid phase thus can be a latex, plastic, derivatized plastic, magnetic or non-magnetic metal, glass or silicon surface or surfaces of test tubes, microtiter wells, sheets, beads, microparticles, chips, and other configurations known to those of ordinary skill in the art.

It is contemplated and within the scope of the invention that the solid phase also can comprise any suitable porous material with sufficient porosity to allow access by indicator reagents. Microporous structures are generally preferred, but materials with gel structure in the hydrated state may be used as well. Such useful solid supports include: natural polymeric carbohydrates and their synthetically modified, crosslinked or substituted derivatives, such as agar, agarose, cross-linked alginic acid, substituted and cross-linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber, synthetic polymers which may be prepared with suitably porous structures, such as vinyl polymers, including polyethylene, polypropylene, polystyrene, polyvinylchloride, polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides; porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and mixtures or copolymers or the above classes, such as graft copolymers obtained by initializing polymerization of synthetic polymers on a pre-existing natural polymer. All of these materials may be used in suitable shapes, such as films, sheets, or plates, or they may be coated onto or bonded or laminated to appropriate inert carriers, such as paper, glass, plastic films, or fabrics.

The porous structure of nitrocellulose has excellent absorption and adsorption qualities for a wide variety of reagents including monoclonal antibodies. Nylon also possesses similar characteristics and also is suitable.

The term "test sample", as used herein, refers to a material suspected of containing analytes. The test sample can be used directly as obtained from the source or following a pretreatment to modify the character of the sample. The test sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid and the like, and fermentation broths cell cultures, and chemical reaction mixtures and the like. In addition to biological or physiological fluids, other liquid samples can be used such as water, food products and the like for the performance of environmental or food production assays. In addition, a solid material suspected of containing an analyte can be used as the test sample. In some instances, it may be beneficial to modify a solid test sample to form a liquid medium or to release an analyte. The test sample can be pretreated prior to use by, for example, preparing plasma from blood, diluting viscous fluids, filtering liquids, distilling liquids, concentrating liquids, inactivating interfering components, adding reagents, and the like. Moreover the test sample can be pretreated to amplify or otherwise concentrate nucleic acid sequences that may be contained therein. Methods of amplification can involve, for example, PCR, LCR, GLCR and the like.

The invention results in large part from the surprising discovery that detectable moieties associated with a common capture reagent can be accurately differentiated from one another based upon the timing at which they generate a signal. This discovery has several important consequences. For example, the presence or amount of multiple analytes in a test sample can be detected based upon the timing with which detectable moieties that are associated with the analytes generate a detectable signal. Moreover, multiple analytes that may be present in a test sample can be indiscriminately immobilized to a common capture reagent without requiring segregation of the analytes into common analyte zones. Accordingly, the method does not require the reagents or equipment necessary for segregating the analytes. Furthermore, multiple detectable moieties that have similar excitation and/or emission wavelengths can be employed in the same assay. Hence, the method does not require multiple detection systems or filters in order to excite and/or detect detectable moieties having multiple excitation and/or emission wavelengths.

Detectable moieties can be separated into classes based upon the types of signals they generate. For example, a "steady state" detectable moiety can be characterized as a detectable moiety that accepts energy through, for example, chemical or physical means and typically emits the energy in the form of light. This phenomenon is variably referred to as excitation and emission. In cases where a steady state detectable moiety accepts energy in, for example, the form of light of a certain wavelength, such a detectable moiety typically emits the energy in the form of light at a wavelength different from the excitation wavelength. Steady state detectable moieties can also be characterized by the period of time between the time they accept energy and the time they emit energy. Typically, this time period is in the nanosecond range and upon excitation, detection of a steady state signal can effectively occur instantaneously. Examples of steady state detectable moieties include, but are not intended to be limited to fluorophores such as coumarin, fluorescein, cascade blue, rhodamine and Texas Red™; p-phthallocyanines; cyanine dyes; thiazoles; dansyl; napthalene; p-toluidinyl napthalene sulfonic acid; phycoerythrin; allophycocyanine; and the like.

Another class of detectable moieties includes "time dependent" detectable moieties. Time dependent detectable moieties can be characterized as a detectable moiety that generates a detectable signal over a period of seconds depending upon the conditions present at the time of signal generation. Consequently, such a signal or the rate at which the signal is generated can be detected over a period of time. An example of a time dependent detectable moiety includes, but is not intended to be limited to an enzyme and its substrate such as, for example, horseradish peroxidase (HRPO) and resorufin, β-D-galactosidase and β-methyl-umbelliferyl-galactoside (MUG), alkaline phosphatase and methyl-umbelliferyl phosphate (MUP), glucourinidase and methyl umbelliferrone glucourinide, and the like.

A further class of detectable moieties includes "time resolved" detectable moieties. Time resolved detectable moieties can be characterized as having a signal which has a microsecond duration and can be detected with a pulsed light source and a detection means that measures the intensity of a signal as a function time whereby the signal's rate of decay is measured. An example of a time resolved detectable moiety includes, but is not intended to be limited to sensitized lanthanide fluorophores such as terbium chelates, europium chelates and samarium chelates.

The detectable moieties employed in the method herein provided are generally selected so that the signals they emit can be differentiated based upon the time(s) at which they are generated and/or the effect or influence one signal has upon another. Although detectable moieties which can be distinguished based upon their emission wavelengths can be employed, preferably, detectable moieties are selected to have common emission wavelengths in order to obviate the need for detection devices that are capable of detecting light at multiple wavelengths. Typically, detectable moieties having a common emission wavelength and a common detectable moiety class can be differentiated from one another by sequentially detecting their signals. Sequentially detecting signals can be accomplished through selectively inhibiting one or more detectable moieties from generating signals whereby signals from a plurality of detectable moieties that are associated with a common capture reagent can be detected one at a time. Consequently, the presence or amount of multiple analytes with which the detectable moieties are associated can be detected one at a time.

Selectively inhibiting a one or more detectable moieties from generating signals can be achieved with inhibitors. Inhibitors that can be employed can be an agent, condition or lack of an agent or condition that can be used to prevent or otherwise control a detectable moiety's ability to generate a signal. For example, a time dependent detectable moiety such as, for example, an enzyme can be prevented from generating a signal by obstructing the enzyme's active site with an appropriate blocking agent. Hence, the enzyme's substrate is prevented from binding, being acted upon, and generating a signal. Alternatively, enzymatic detectable moieties can be selected so that they have activity in certain pH ranges but not in others. Thus, the generation of individual signals can be selectively controlled by varying pH levels. Moreover a chelating agent can be employed to bind an enzyme's cofactor and thereby prevent enzymatic activity and signal generation. Further, depriving an enzyme of its substrate can be used to inhibit the enzyme's action. An inhibitor can also take the form of allowing one enzyme's activity to plateau before adding a different enzyme's substrate which results in a distinguishable rate of signal generation. Additionally, a steady state detectable moiety or a time resolved detectable moiety can be inhibited by depriving it of its excitation energy. Table 1, below, provides examples of detectable moieties, the class to which the detectable moiety belongs, and examples of inhibitors for the detectable moieties.

**Table 1**

| **Detectable Moiety** | **Detectable Moiety Class** | **Inhibitors** |
|---|---|---|
| Alkaline Phosphatase | Time Dependent | Chelating agents - ethylene diamine tetraacetic acid (EDTA) and ethylene diamine pentaccetic acid (DPTA); Substrate deprivation - MUP deprivation; Active site blockers - anti-alkaline phosphatse antibodies; |
| β-D-galactosidase | Time Dependent | Chelating agents - ethylene diamine tetraacetic acid (EDTA) Substrate deprivation - MUG deprivation; Active site blocks - phenyl ethyl β-D-thiogalactopyranoside, and anti-galactosidase antibodies |
| polycoumarin on a polylysine polymer | Steady State | - |
| poly 4-methylumbelliferrone-3-acetic acid on a polylysine polymer | Steady state | - |

For example, in cases where a detectable moiety from one class, for example a fluorophore, and multiple detectable moieties from another class, for example enzymes having fluorogenic substrates, are employed, the enzymatically produced signals can be detected sequentially by selectively inhibiting enzymatic action. Inhibiting enzymatic action will also inhibit signal generation. Specifically, after a plurality of enzymes are associated with a plurality of analytes, the enzymes can be contacted with a substrate for one enzyme and inhibitors for the other enzymes. At this point the inhibition can be, for example, in the form of substrate deprivation whereby the immobilized enzymes can be contacted with a substrate for a single enzyme. Accordingly, one enzyme will generate a signal while the others are prevented from generating a signal. Hence, the presence or amount of the analyte associated with the uninhibited enzyme can be detected. After the first signal has been detected, the reaction mixture can be contacted with another combination of substrate and inhibitors whereby a signal from a different enzyme, and therefore the presence or amount of a different analyte, can be detected. Accordingly, the enzyme that was initially producing a signal as well as all but one of the other enzymes can be inhibited by contacting the enzymes with a substrate for one of the other enzymes and a chelating agent for the first enzyme which prevents the first enzyme from further signal generation. Hence, the first enzyme is inhibited by the chelating agent and all but one of the other enzymes continue to be inhibited due to a lack of substrate. After the signal from the second enzyme has been detected, the second enzyme can be permitted to reach its equilibrium and the rate of the signal generated by the second enzyme will consequently level off or plateau and therefore be inhibited. When this equilibrium is reached, the enzymes can be contacted with a substrate for a third enzyme and inhibitors for the remaining enzymes. For example, the enzymes can be contacted with the third enzymes substrate in a suitable buffer having a pH wherein the third enzyme has activity and other enzymes which have not been contacted with their substrate do not As a result, a third signal can be determined and the presence or amount of a third analyte in the test sample can also be determined. By contacting the enzymes with a fourth substrate in a suitable buffer having a pH wherein the fourth enzyme has activity but the others do not, a fourth signal can be detected as an indication of the presence or amount of a fourth analyte in the test sample. By continuing in this manner, a plurality of individual signals from a single detectable moiety class can sequentially be detected and differentiated. Hence, a plurality of analytes that may be present in a sample can be detected. It will be understood, of course, that the method employed to inhibit a detectable moiety's ability to generate a signal and the order in which detectable moieties are inhibited are not intended to limit the invention.

While distinct classes of detectable moieties which have common emission wavelengths can be differentiated by sequentially detecting their signals as outlined above, distinct classes of detectable moieties which have a common emission wavelength can alternatively be differentiated by detecting their signals at substantially the same time. For example, in the case where a steady state detectable moiety such as, for example, a fluorophore, and a time dependent detectable moiety such as, for example, an enzyme and its fluorogenic substrate are employed, the enzyme can be deprived of its substrate while the signal from the fluorophore is detected. The enzyme can then be contacted with its substrate to produce a time dependent signal which then can be detected. Hence, the signals generated by the detectable moieties are detected sequentially.

Alternatively, the enzyme can be contacted with its substrate at the same time the signal generated by the fluorophore is being detected. At the instant of such contact, time zero, the steady state detectable moiety can generate a signal which can be detected instantaneously. Additionally, at time zero, the time dependent detectable moiety will have generated a negligible signal because it has not had sufficient time to convert its substrate and thereby generate a detectable signal. However, shortly after time zero, the time dependent signal can be detected. Hence, while the signals are generated at substantially the same time, they can nevertheless be distinguished without the necessity of an inhibitor in the following manner. Preferably, such signals are detected periodically over a period of time and a linear least square fit is performed on the measured signals. The slope of the resulting line is used as the time dependent signal and the intercept is used as the steady state signal. It will be understood, of course, that other combinations and numbers of detectable moieties can be differentiated based upon the timing with which they generate a signal and are contemplated as within the scope of the invention. It will also be understood that in cases where the detectable moieties generate a signal having a common wavelength, the initial signal generated from a steady state detectable moiety or time resolved detectable moiety, for example, can be subtracted from or added to a signal generated by a time dependent detectable moiety to arrive at the amount of signal attributable exclusively to the time dependent detectable moiety. Hence, the influence of the steady state detectable moiety or time resolved detectable moiety upon the time dependent signal can be used to differentiate the signals.

According to the present invention, multiple analytes that may be present in a test sample are preferably immobilized to a common capture reagent. Thus, a capture reagent according to the instant invention can comprise multiple binding members which form binding pairs with multiple analytes or a single binding member that forms a binding pair with multiple analytes that may be present in a test sample. In any case, the capture reagent will be capable of immobilizing at least two analytes. Preferably, a solid phase comprises a suspension of microparticles coated with a single binding member or multiple binding members which form binding pairs with multiple analyzes that may be present in a test sample. While many different analytes can form a binding pair with a common or single binding member, it may be necessary to modify analytes to allow them to form a binding pair with a single binding member. For example, while follicle stimulating hormone (FSH) and leutinizing hormone (LH) share a common subunit and can therefore be immobilized to a capture reagent comprising a single binding member that binds the common subunit, not all analytes share this characteristic. In cases where multiple analytes do not inherently form binding pairs with a single capture reagent, an ancillary binding member or binding members can be employed.

An ancillary binding member can be capable of binding one or more analytes to the capture reagent's binding member. Hence, an ancillary binding member can be selected to form binding pairs with an analyte and the capture reagent's binding member. It will be understood of course that those skilled in the art will recognize many methods for immobilizing multiple analytes to a capture reagent and that the manner in which analytes are bound to a capture reagent is not intended to limit the invention.

Embodiments of the invention will now be discussed in conjunction with the drawings which graphically illustrate time differentiated detection of multiple analytes that may be present in a test sample.

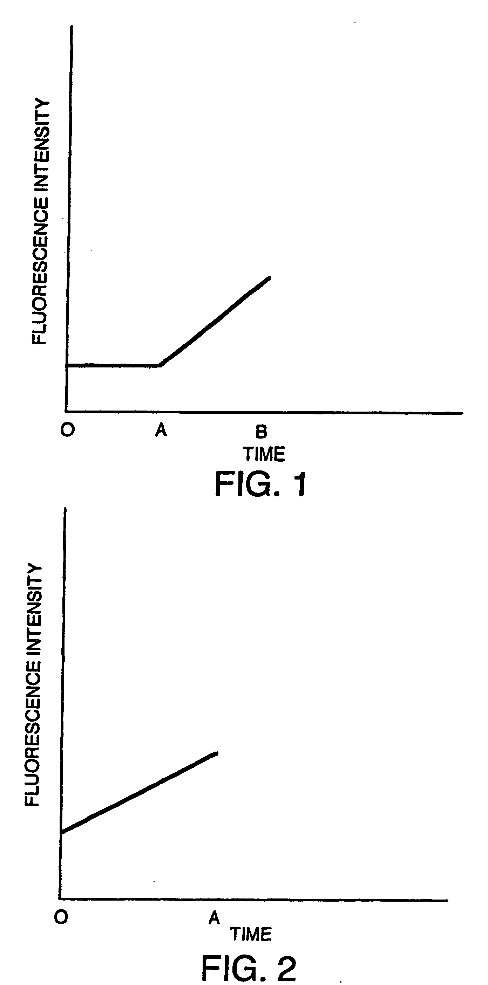

According to one embodiment, the presence or amount of a first and a second analyte which may be present in a test is detected using a steady state detectable moiety and time-dependent detectable moiety. In accordance with this embodiment, a test sample is contacted with a capture reagent, which comprises a solid phase coated with a binding member or binding members that form binding pairs with the analytes that may be present in the test sample, for a time and under conditions sufficient to form capture reagent/first analyte complexes and capture reagent/second analyte complexes. The capture reagent, and the complexes immobilized thereon, can then be removed from the excess test sample and the complexes can be contacted with an indicator solution comprising two indicator reagents. A first indicator reagent can comprise a binding member specific for a first analyte labeled with a steady state detectable moiety, and a second indicator reagent can comprise a binding member specific for a second analyte labeled with a time dependent detectable moiety such as, for example, an enzyme. The indicator solution is contacted with the capture reagent/analyte complexes for a time and under conditions sufficient to form capture reagent/first analyte/first indicator reagent complexes and capture reagent/second analyte/second indicator reagent complexes. The steady state fluorescence intensity then can be measured by integrating or averaging the fluorescence signal over several short time intervals such as, for example, successive measurements of 500 µs duration at 700 µs intervals. The mean of these measurements can be used to indicate the presence or amount of a first analyte in the test sample. As shown by Figure 1, such a steady state signal is shown between time zero and time A. A substrate for the time dependent detectable moiety can then be contacted with the capture reagent/analyte/indicator reagent complexes. A rate of fluorescent signal generation can then be measured by detecting the fluorescent signal over several short time intervals such as, for example, successive measurements of 500 µs duration at 700 µs intervals. The fluorescent intensity as a function of time can be plotted to produce a line the slope of which corresponds to the rate of fluorescent signal generation. This rate can then be used as an indication of the presence or amount of a second analyte in the test sample. As illustrated by Figure 1, at time A the time dependent detectable moiety's substrate is contacted with the capture reagent/analyte/indicator reagent complexes and a time dependent signal is generated between time A and time B. This time dependent signal can then be used to indicate the presence or amount of a second analyte in the test sample. Hence, the presence or amount of both analytes is determined sequentially.

Alternatively, the presence or amount of both analytes in the test sample can be determined simultaneously by contacting the capture reagent/analyte/indicator reagent complexes with the time dependent detectable moiety's substrate and simultaneously detecting two signals as an indication of the presence or amount of both analytes in the test sample. As shown by Figure 2, at time zero the capture reagent/analyte/indicator reagent complexes are contacted with the time dependent detectable moiety's substrate and a line corresponding to the time dependent signal can be plotted starting at time zero. The y-intercept of the fluorescence intensity v. time line can be used as the fluorescent signal generated by the steady state delectable moiety and therefore the presence or amount of a first analyte in the test sample. The slope of the line can be used to indicate the presence or amount of a second analyte in the test sample. In cases exemplified by Figure 2, the influence of the steady state signal locates the y-intercept of the fluorescence intensity v. time line. Hence, the presence or amount of both analytes can be determined using detectable moieties having the same excitation and emission wavelengths by sequentially or simultaneously detecting the steady state and time dependent signals as an indication of the presence or amount of two analytes in a test sample.

According to another embodiment, a steady state detectable moiety and two time dependent detectable moieties can be employed to detect a first analyte, a second analyte and a third analyte that may be present in a test sample. Similarly to above embodiments, a test sample is contacted with a capture reagent, which comprises a solid phase coated with a binding member or binding members that form binding pairs with the analytes that may be present in the test sample, for a time and under conditions sufficient to form capture reagent/first analyte complexes, capture reagent/second analyte complexes and capture neagent/third analyte complexes. The so-formed complexes then can be contacted with an indicator solution comprising three indicator reagents for a time and under conditions sufficient to form capture reagent/analyte/indicator reagent complexes. A first indicator reagent can comprise a binding member specific for a first analyte labeled with a steady state detectable moiety; a second indicator reagent can comprise a binding member specific for a second analyte labeled with a time dependent detectable moiety such as, for example, a first enzyme having a first fluorogenic substrate; and a third indicator reagent can comprise a binding member specific for a third analyte labeled with a time dependent detectable moiety such as, for example, a second enzyme having a second fluorogenic substrate. Similarly to an earlier embodiment, the signal generated by the steady state detectable moiety and any one of the time dependent detectable moieties can be detected simultaneously or all of the signals can be detected sequentially. The signal from the steady state signal can be used as a measure of the presence or amount of a first analyte in the test sample. The signal generated by the first enzyme can be detected by contacting the capture reagent/analyte/indicator reagent complexes with a first substrate and determining a rate of conversion as an indication of the presence or amount of a second analyte. After sufficient time has elapsed the rate of the first enzymes signal generation will plateau or level off and accordingly be inhibited; and a second substrate can be contacted with the capture reagent/analyte/indicator reagent complexes. The second enzyme will generate a new rate of signal generation which can then be used to indicate the presence or amount of a third analyte that may be present in the test sample.

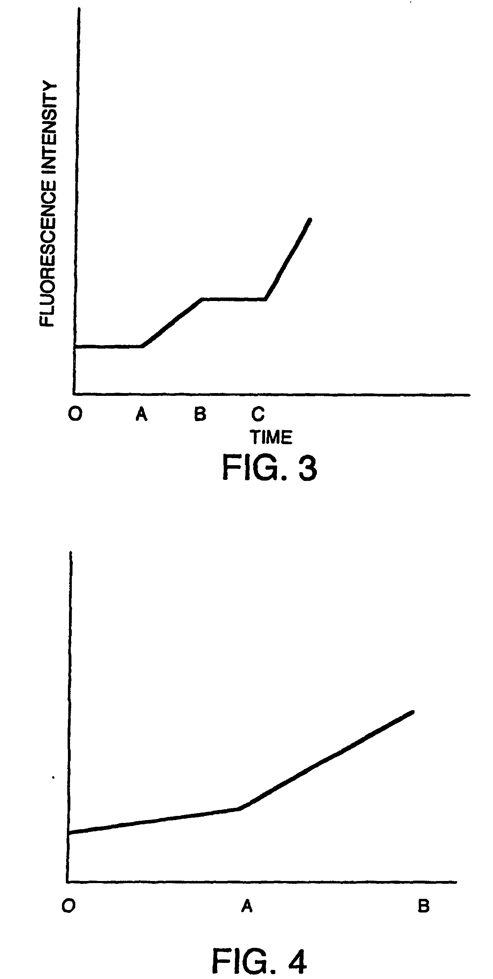

Figure 3, illustrates a graph of fluorescence intensity v. time which is consistent with the immediately preceding embodiment. Figure 3, graphically depicts the sequential detection of three analytes which may be present in a test sample using a steady state detectable moiety and two time dependent detectable moieties. As shown in Figure 3, from time zero to time A, a steady state signal can be detected as an indication of the presence or amount of a first analyte. At time A, a substrate can be contacted with capture reagent/analyte/indicator reagent complexes and a time dependent signal begins and runs to time B at which point the signal plateaus. The rate signal between time A and time B can be used to indicate the presence or amount of a second analyte in a test sample. Between time B and time C, the activity of the first time dependent detectable moiety has plateaued and the signal generated thereby has also plateaued. At time C, the capture reagent/analyte/indicator reagent complexes can be contacted with the second time dependent detectable moiety's substrate and a new rate of signal generation will begin. This signal can then be used to indicate the presence or amount of a third analyte that may be present in the test sample.

Alternatively, rather than allowing the first time dependent signal to plateau after a signal was detected, it would be possible to inhibit the first time dependent detectable moiety in another manner which would cause a substantially complete cessation of signal generation. Accordingly, the rate of signal generated by the second time dependent detectable moiety could be calculated from the baseline created by the presence of the steady state detectable moiety and the presence of the first detectable moiety's product

For example, after the capture reagent first analyte, second analyte and third analyte complexes are formed, the so-formed complexes can be contacted with an indicator solution for a time and under conditions sufficient to form capture reagent/analyte/indicator reagent complexes. The indicator solution may comprise three indicator reagents. A first indicator reagent can comprise a binding member specific for a first analyte labeled with a steady state detectable moiety. A second indicator reagent can comprise a binding member specific for a second analyte labeled with a time dependent detectable moiety such as, for example, a first enzyme having activity at an acidic pH but substantially no activity at an alkaline pH; and a third indicator reagent can comprise a binding member specific for a third analyte labeled with a time dependent detectable moiety such as, for example, a second enzyme having activity at an alkaline pH but substantially no activity at an acidic pH. The capture reagent/analyte/indicator reagent complexes can then be contacted with the first enzyme's substrate in an appropriate buffer comprising an inhibitor such as, for example, an acidic pH. At this pH, the second enzyme is inhibited, but the first will be active and produce a time dependent signal. The rate of such signal production can be used to indicate the presence or amount of a first analyte in the test sample. After a first signal has been detected, the capture reagent/analyte/indicator reagent complexes can be contacted with the second enzyme's substrate in an appropriate buffer comprising an inhibitor such as, for example, an alkaline pH. At this pH range, the first enzyme is inhibited, but the second enzyme will be active and produce a time dependent signal. The rate of this signal production can be used to indicate the presence or amount of a second analyte in the test sample.

Figure 4, illustrates a graph of fluorescence intensity v. time which could result from the simultaneous and sequential detection of multiple analytes using a steady state detectable moiety and two time dependent detectable moieties. As shown in Figure 4, at time 0, when a substrate for the first time dependent detectable moiety and an inhibitor for the second time dependent detectable moiety is contacted with the capture reagent/analyte/indicator reagent complexes, a steady state signal can be detected to indicate the presence or amount of a first analyte in the test sample. Additionally, the first time dependent signal begins to develop at time 0. The signal from the first time dependent signal continues to develop until time A when an inhibitor for the first time dependent detectable moiety and a substrate for the second time dependent detectable moiety is contacted with the capture reagent/analyte/indicator reagent complexes. The signal generated between time 0 and time A can be used to indicate the presence or amount of second analyte that may be present in the test sample. Also at time A, the second time dependent detectable moiety begins to generate another signal which is allowed to develop sufficiently, until time B, to allow detection of the presence or amount of a third analyte that may be present in the test sample.

In a preferred embodiment of the invention, the method herein provided is employed to detect the presence or amount of at least two nucleic acid sequences that may be present in a test sample. Accordingly, the analytes comprise nucleic acid sequences or are otherwise the products of a hybridization reaction such as LCR which is described in European Patent Applications EP-A-320-308 and EP-A-439-182 and PCR which is described in U.S. Patents Numbered 4,683,202 and 4,683,195 all of which are herein incorporated by reference.

In cases where the analytes comprise, for example, LCR or PCR reaction products or sequences, the sequences can comprise or be modified to comprise a reporter region and a capture region. The reporter region can comprise a nucleotide or several nucleotides which have been labeled with a detectable moiety. Alternatively, the reporter region can comprise a binding member that forms a binding pair with an indicator reagent The capture region can comprise a binding member which forms a binding pair with the capture reagent. Hence, sequences can be immobilized to a capture reagent via capture regions and the presence or amount of any immobilized sequences can be determined via the reporter region.

For example, the method herein provided can be employed to detect the presence or amount of a first amplified nucleic acid sequence and a second amplified nucleic acid sequence that may be present in a test sample. The first and second sequences can have a capture region comprising a common hapten which forms a binding pair with a capture reagents binding member. Additionally, the first and second sequences can have a reporter region comprising (i) a hapten which forms a binding pair with an indicator reagent or (ii) one or more nucleotides which are labeled with a detectable moiety. In the latter case, the analyte itself will comprise an indicator reagent in the form of the labeled nucleotides. The test sample comprising the amplified nucleic acid sequences, if any, can be contacted with a capture reagent, which preferably comprises a suspension of microparticles that are coated with a binding member that forms a binding pair with the common haptens, for a time and under conditions sufficient to form capture reagent/sequence complexes. The so-formed complexes then can be contacted with at least one indicator reagent for a time and under conditions to further form capture reagent/sequence/indicator reagent complexes. Such complexes preferably comprise at least one sequence which is labeled with a steady state detectable moiety and at least one sequence which is labeled with a time-dependent detectable moiety. A time differentiated signal can then be detected, as outlined above, as an indication of the presence or amount of at least two sequences in the test sample.

In another particularly preferred embodiment, one of the analyzes to be detected can be a positive control which ensures that the reagents and methods employed to perform an amplification assay are efficacious. For example, in the case of an amplification assay designed to amplify a target sequence that may or may not be present in a test sample, a sequence that is known to exist in the test sample can be amplified and detected as taught herein to ensure that the amplification procedure itself was completed effectively.

As previously mentioned, the method of the present invention can be adapted for use in automated systems. Such systems are, for example, described in U.S. Patent Application No. 425,643 which corresponds to European Patent Application No. EP 0 424 634. A particularly preferred and commercially available automated instrument which can be employed to perform the method herein provided is the IMx® system which is available from Abbott Laboratories, Abbott Park, IL. Protocols for Microparticle Enzyme ImmunoAssays (MEIAs) such as those performed by the Abbott IMx® instrument are well known in the art and have been described in Fiore, M. *et al., Clin. Chem*., **34**:1726-1732 (1988). An exemplary protocol is as follows. 100µl of a test sample is pipetted into the sample well of an IMx® reaction cell. 30-50µl of the sample and an anti-analyte coated micropartide suspension are then pipetted into the reaction cell's incubation well. An appropriate incubation period follows which allows the formation of microparticle/analyte complexes. The complexes are then pipetted onto the reaction cell's glass fiber capture matrix and a conjugate comprising an anti-analyte antibody labeled with an enzyme is also pipetted onto the reaction cell's glass fiber matrix. Microparticle/analyte/conjugate complexes are formed and captured by the glass fiber matrix. A substrate for the conjugate's enzyme is then pipetted onto the glass fiber matrix and the enzyme converts the substrate into a fluorescent product. The rate of production of the fluorescent product is then measured, and the rate of such production which is expressed in counts/second/second (c/s/s) is directly related to the amount of analyte in the test sample.

It will be understood of course that while the method herein described can detect the presence or amount of multiple analyzes that may be present in a test sample, one or more of the analytes may not be present in a particular test sample. Accordingly, the absence of signal generated by one or more individual detectable moieties is nevertheless a detection of the presence or amount of an analyte or analytes in a test sample. Additionally, it is contemplated and within the scope of the invention that washing can occur between any or all the steps of the method herein disclosed.

The following examples are provided to further illustrate the present invention and not intended to limit the invention. Unless otherwise specified, all reagents and equipment used in the following examples are commercially available. In all of the examples where applicable, eight readings per detectable moiety were made. In cases where steady state detectable moieties were employed, the average of the eight readings was used as the signal. In cases where time-dependent detectable moieties were employed, linear regression was performed on the eight readings to obtain a slope which was indicative of the rate of signal generation. Additionally, where applicable, a intercept could also be calculated to indicate a steady state signal.

### Example 1

### Detection of a Time Dependent Signal in the Presence of a Steady State Signal

This example demonstrates the detection of a signal generated by a time dependent detectable moiety in the presence and absence of a signal generated by a steady state detectable moiety. The steady state signal was generated by a suspension of fluorescent microparticles and the time dependent signal was generated by alkaline phosphatase enzyme and its fluorogenic substrate methyl-umbelliferyl phosphate (MUP).

### Materials:

1. Fluorescent Mircorparticle Solution - 1.7µm-2.2µm diameter Light-yellow high-intensity fluorescent microparticles (Spherotech Inc., Libertyville, IL) having an average diameter of 2 µm and a concentration of 1 (w/v) were diluted in 0.02 M tris® 0.03 M tris® hydrochloride, 0.1 M sodium chloride, 13.6% sucrose, 0.1% sodium azide buffer pH 8.0 to a weight concentration of 0.025% (w/v).
2. Anti-Carbazole Labeled Microparticle Solution - A solution of anti-carbazole antibody labeled microparticles was prepared by coupling polyclonal rabbit anti-carbazole antibody to carboxylated polystyrene microgarticles using known (1-ethyl 3-(3-dimethylaminopropyl) carbodiimide (EDAC) coupling procedures. See Brown, *Clin. Chem.,* **33**:1567-1568 (1987).
3. Mixed Microparticle Solution - The mixed microparticle solution comprised 1:1 mixture of the fluorescent particles and anti-carbazole labeled microparticles.
4. Anti-Adamantane Conjugate - The conjugate comprised a polyclonal anti-adamantane antibody attached to alkaline phosphatase enzyme. The conjugate was prepared using standard EDAC coupling procedures. The conjugate was diluted to 0.01 µg/ml in 0.02 M tits®, 0.13 M tris® hydrochloride, 0.02 M magnesium chloride, 0.1 nM zinc chloride, 0.4 M sodium chloride, 10% mannitol, 3% BRIJ® 35 (Calbiochem®; LaJolla, CA), [See Ashani, Y., *et al., Anal. Biochem*., **109**: 55 (1980)] 3% BSA, ∼ 0.1% sodium azide buffer pH ∼8.0
5. Substrate - 1.2 mM methyl-umbelliferyl phosphate (MUP, available from Abbott Laboratories) in a buffered solution having a pH of 10.1 was used as a substrate.
6. Controls - The controls comprised three dilutions of a synthetic oligonucleotide (SEQ ID No. 1) which was covalently linked to carbazole at the 5' terminal nucleotide and covalently linked to adamantane at the 3' terminal nucleotide. This sequence was designated C-30-A and the carbazole was used as the capture hapten and the adamantane was used as the reporter hapten. A stock solution of 1.0 x 10¹³ molecules/ml of the C-30-A sequence was prepared in 50 mM Epps buffer and 0.01% BRIJ® 35. The three dilutions were prepared from the stock solution so as to give a specified signal (rate count/s/s) on the IMx® when used with the capture microparticles and the conjugate. The dilutions were relative to each other and designated "low", "medium" and "high" to indicate the relative amount of the sequence in the sample.

### Method:

An IMx® system (available from Abbott Laboratories) was used to run two assays. In the first assay the anti-carbazole labeled microparticles were placed in the microparticle bottle, the anti-adamantane conjugate was placed in the conjugate bottle, and MUP was placed in the substrate bottle of an IMx® system's reagent pack. Assays were run in the absence of a control as well as in the presence of the low, medium and high controls by programming the IMx® system to execute the following steps:
1. 50 µl of the microparticles from the microparticle bottle and 50 µl of the control, if used, were dispensed into the incubation well and allowed to incubate for 8.6 minutes;
2. 125 µl of the control/microparticle mixture or microparticle mixture was then transferred to the glass fiber matrix;
3. 50 µl of the conjugate was then dispensed onto the glass fiber matrix and allowed to incubated for 5 minutes;
4. 60 µl of substrate was then dispensed onto the matrix and the rate of fluorescence generation was measured for 500 µsec intervals for a period of 700 µsec.

Accordingly, the rate of fluorescence production in the absence of a steady state signal was determined in the presence and absence of the controls. This data can be found below in Table 2 under "Assay #1".

A second assay was run in the same manner as the first assay, except the mixed microparticle solution was placed in the microparticle bottle of the IMx® system's reagent pack. Similarly to the first assay the rate of fluorescence production was determined in the presence and absence of the controls. However, in this assay, the measurements were determined in the presence of a steady state signal because of the presence of the fluorescent microparticles. The data for this assay is also shown below in Table 2 under "Assay #2".

**Table 2**

| **Rate of Fluorescence Generation** | | | | |
|---|---|---|---|---|
| | Assay #1 | | Assay #2 | |
| | Mean Rate | | Mean Rate | |
| | (c/s/s) | % CV | (c/s/s) | % CV |
| No Control | 8 | 10 | 8 | 10 |
| Low | 403 | 2.07 | 406 | 1 |
| Medium | 757 | 2.51 | 765 | 1.4 |
| High | 876 | 2.0 | 885 | 2.04 |

The data indicate that a fluorescence rate signal is not affected by the presence of a steady state fluorescence signal. Additionally, by calculating the linear regression of the rate, the intercept, which represents the background fluorescence, can be determined. In the presence of the fluorescent microparticles, the intercept/background fluorescence represents the steady state fluorescence signal. The intercept data for the two assays is shown below in Table 3 where "Assay #1" corresponds to the assay run without fluorescent microparticles and "Assay #2" corresponds to the assay run with fluorescent microparticles. The difference in the mean intercept between the two assays (due to the presence of the steady state fluorescence signal) is shown in the last column of Table 3 as "Δ Intercept".

**Table 3**

| | Assay #1 | | Assay #2 | | |
|---|---|---|---|---|---|
| | Mean Intercept | % CV | Mean Intercept | % CV | Δ Intercept |
| No Control | 1654 | 0.7 | 3149.7 | 1.46 | 1495.5 |
| Low | 2008.65 | 1.1 | 3533.6 | 0.78 | 1529.9 |
| Medium | 2288.67 | 2.3 | 3918.4 | 1.1 | 1629.7 |
| High | 2521.7 | 5.8 | 4037.25 | 1.3 | 1515.6 |

The mean increase in intercept due to the presence of the fluorescent microparticles is 1542.68 counts (CV = 3.87%). This data shows the ability to detect a fluorescence rate signal super-imposed on a steady-state fluorescence signal and conversely the ability to detect a steady state signal in presence of a rate signal without affecting the precision of either measurement.

### Example 2

### Simultaneous Detection of Two Analytes Using Two Time Dependent Detectable Moieties

### Materials:

1. Test Sample - The test sample comprised a mixture of two analytes. The first analyte, SEQ. ID. No. 1, was the 30 nucleotide nucleic acid sequence which is designated C-30-A. The C-30-A sequence had a carbazole capture hapten and an adamantane reporter region. The second analyte, designated B-15-F, is a 15 carbon straight chain alkyl compound which is available from Molecular Probes, Eugene, OR. The B-15-F had a fluorescein capture region and a biotin reporter region.
2. Capture Reagent - The capture reagent employed in this example was a suspension of and-carbazole antibody coated microparticles and and-fluorescein labeled microparticles both of which were prepared using conventional EDAC coupling procedures. The particles were suspended in 0.02 M tris®, 5.4 gm/L sodium chloride, 13.6% sucrose, 1% sodium azide buffer pH 8.0.
3. Indicator Solution - The indicator solution employed in this example was a mixture of and-adamantane antibody labeled with alkaline phosphatase enzyme and avidin labeled with β-D-galactosidase enzyme. The alkaline phosphatase conjugate was prepared using standard EDAC coupling methods and the avidin β-D-galactosidase conjugate is available in a lyophilized form from Boehringer Mannheim, Indianapolis IN. The β-D-galactosidase was reconstituted in 0.02 M tris®, 0.13 M trimethane, 0.02 M magnesium chloride, 0.1 nM zinc chloride, 0.4 M sodium chloride, 10% mannitol, 3% BRIG-35, 3% BSA ∼0.1% sodium azide buffer to form a 1.46 IU/ml solution. The indicator solution was made by mixing 3.0 ml of alkaline phosphatase conjugate with 8.8 ml of the β-D-galactosidase conjugate.
4. Substrates - Methyl umbelliferyl phosphate (MUP) solution (pH 10.1), available from Abbott Laboratories, was used as the alkaline phosphatase conjugate's substrate. β-methyl-umbelliferyl-galactoside (MUG), in a lyophilized form was purchased from Molecular Probes Inc., Eugene OR and was used as the β-D-galactosidase enzyme's substrate. Prior to use, MUG was first dissolved in dimethyl sulfoxide (DMSO) to yield a 34 mg/ml solution and further diluted in 0.1 M tris®-acetate buffer (pH 7.5) to yield a final MUG concentration of 0.34 mg/ml.
5. Wash Buffer - The wash buffer used in this example was 0.1 M tris®-acetate pH 7.1.

### Procedure:

Using an IMx® system, six assays for various concentrations of C-30-A and B-15-F in a test sample were performed. The test sample was diluted such that the individual concentrations of C-30-A and B-15-F varied from test sample to test sample. The dilutions were made in a relative fashion such that the concentrations of sequences in the test samples were either low medium or high with respect to one another.

The IMx® system's reagent pack was configured such that the capture reagent was placed in the microparticle bottle position and the indicator solution was placed in the conjugate bottle position. The substrate solutions were individually dispensed in dilution cups of IMx® test wedges and the various test samples were placed in the sample wells of the test wedges. The test wedges were assembled in the IMx® system's carousel and the IMx® was programmed such that it executed the following steps:
1. 50 µl of the microparticle mixture from the microparticle bottle and 50 µl of the test sample were dispensed into the incubation well and allowed to incubate for 8.6 minutes;
2. 125 µl of the sample/microparticle mixture was then transferred to the glass fiber matrix and washed with 150 µl of wash buffer;
3. 50 µl of the indicator reagent was then dispensed onto the glass fiber matrix and allowed to incubated for 5 minutes;
4. the glass fiber matrix was then washed with 69 µl of wash buffer,
5. 60 µl of MUG substrate was then dispensed onto the matrix and the rate of fluorescence generation was measured for 500 µsec intervals for a period of 700 µsec;
6. the glass fiber matrix was then washed with 150 µl of wash buffer to partially wash the MUG substrate from the matrix;
7. then 60 µl of MUP substrate was dispensed onto the matrix and the rate of fluorescence generation was measured for 500 µsec intervals for a period of 700 µsec.

The rate data for the various assays are shown below in Table 4. As shown by the data, two different rate signals can be distinguished and employed to indicate the concentration of two analytes in a test sample.

**Table 4**

| | **MUG Substrate** | | **MUP Substrate** | |
|---|---|---|---|---|
| Test Sample | Mean Rate | | Mean Rate | |
| (C-30-A/B-15-F) | (c/s/s) | % CV | (c/s/s) | % CV |
| Negative/Negative | 7 | 4.3 | 8 | 12.5 |
| Low/Low | 37 | 2.7 | 69 | 5.8 |
| Medium/Medium | 150 | 2.8 | 142 | 1.6 |
| High/High | 225 | 2.7 | 211 | 2.5 |
| High/Negative | 224 | 2.7 | 45 | 8.4 |
| Negative/High | 7 | 4.9 | 167 | 4.2 |

### Example 3

### Simultaneous Detection of HIV and Chlamydia Trachomatis Nucleic Acid Sequences

### Materials:

1. Test Sample - The test sample comprised a mixture of two analytes. The first analyte was an LCR amplification product from *Chlamydia trachomatis* and is designated SEQ. ID. No. 2 (hereinafter the chlamydia sequence). The chlamydia sequence had a 5' carbazole hapten capture region and a 3' adamantane hapten detection region. The second analyte was an LCR amplification product of HIV which is designated SEQ. ID. No. 3 (hereinafter the HTV sequence). The HIV sequence had a 5' fluorescein hapten capture region and a 3' biotin hapten detection region.
2. Capture Reagent - The capture reagent employed in this example was a 1:1 suspension of polyclonal rabbit anti-carbazole antibody coated microparticles and polyclonal rabbit anti-fluorescein labeled microparticles at a concentration of 0.025% solids. The microparticles were coated using standard EDAC coupling techniques and suspended in 0.02 M tris®, 5.4 gm/L sodium chloride, 13.6% sucrose, 0.1% sodium azide buffer pH 8.0 .
3. Indicator Solution - The indicator solution employed in this example was a mixture of anti-adamantane antibody labeled with alkaline phosphatase and avidin labeled with β-D-galactosidase. The alkaline phosphatase conjugate was prepared using standard EDAC coupling methods and the β-D-galactosidase conjugate is available in a lyophilized form from Boehringer Mannheim, Indianapolis IN. The β-D-galactosidase was reconstituted in 0.02 M tris®, 0.13 M trimethane, 0.02 M magnesium chloride, 0.1 nM zinc chloride, 0.4 M sodium chloride, 10% mannitol, 3% BRIG-35, 3% BSA, ∼0.1% sodium azide buffer to form a 1.62 IU/ml solution. The indicator solution was made by mixing 3.0 ml of alkaline phosphatase conjugate with 8.8 ml of the β-D-galactosidase conjugate.
4. Substrates - The substrates used in this example were the same as those used in Example 2.
5. Wash Buffer - The wash buffer used in this example was the same as that used in Example 2.

### Procedure:

Using an IMx® system, eight assays for various concentrations of the chlamydia sequence and the HIV sequence in a test sample were performed. The sequences were combined to form test samples such that the individual concentrations of the sequences varied from test sample to test sample. The amount of the sequences that were contained in the test samples were relative to each other.

The IMx® system's reagent pack was configured such that the capture reagent was placed in the microparticle bottle position and the indicator solution was placed in the conjugate bottle position. The substrate solutions were individually dispensed in dilution cups of IMx® test wedges and the various test samples were placed in the sample wells of the test wedges. The test wedges were assembled in the IMx® system's carousel and the IMx® was programmed such that it executed the following steps:
1. 50 µl of the microparticle mixture from the microparticle bottle and 50 µl of the test sample were dispensed into the incubation well and allowed to incubate for 8.6 minutes;
2. 125 µl of the sample/microparticle mixture was then transferred to the glass fiber matrix and washed with 150 µl of wash buffer;
3. 50 µl of the indicator reagent was then dispensed onto the glass fiber matrix and allowed to incubated for 5 minutes;
4. the glass fiber matrix was then washed with 69 µl of wash buffer,
5. 60 µl of MUG substrate was then dispensed onto the matrix and the rate of fluorescence generation was measured for 500 µsec intervals for a period of 700 µsec;
6. the glass fiber matrix was then washed with 150 µl of wash buffer to partially wash the MUG substrate from the matrix;
7. then 60 µl of MUP substrate was dispensed onto the matrix and the rate of fluorescence generation was measured for 500 µsec intervals for a period of 700 µsec.

The rate data for the various assays is shown below in Table 5. As shown by the data, two different rate signals can be sequentially detected to indicate the presence or amount of at least two analytes in a test sample.

**Table 5**

| | **MUG Substrate** | | **MUP Substrate** | |
|---|---|---|---|---|
| Test Sample | Mean Rate | | Mean Rate | |
| (Chlamydia/HIV) | (c/s/s) | % CV | (c/s/s) | % CV |
| Negative/Negative | 5.5 | 11.4 | 8.6 | 11.2 |
| Negative/Low | 7.3 | 8.0 | 123 | 1.4 |
| Negative/High | 6.9 | 6.3 | 342 | 2.1 |
| Low/Negative | 126 | 2.6 | 32 | 13.1 |
| Low/Medium | 113 | 2.0 | 248 | 1.9 |
| High/Negative | 214 | 0.8 | 48 | 31.3 |
| High/Low | 216 | 3.3 | 182 | 4.0 |
| High/High | 219 | 0.3 | 374 | 0.9 |

While the invention has been described in detail and with reference to specific embodiments thereof, it will be apparent to one skilled in the art that various changes and modifications may be made therein without departing from the scope thereof.

## Claims

1. A method for determining the presence or amount of at least two analytes that may be present in a test sample, said method comprising the steps of:
(a) contacting said test sample with a common capture reagent for a time and under conditions sufficient to form capture reagent/analyte complexes wherein said common capture reagent includes one or more specific binding members that bind at least two analytes that may be present in said test sample;
(b) contacting said capture reagent/analyte complexes with at least two indicator reagents for a time and under conditions sufficient to form capture reagent/analyte/indicator reagent complexes wherein at least one of said at least two indicator reagents comprises an enzymatic label and at least one of said at least two indicator reagents comprises a fluorophore; and
(c) detecting at least two measurable signals as a measure of the presence or amount of said analytes in said test sample.

2. The method of claim 1 wherein said signals are differentiated based upon the influence one of said signals has upon another of said signals; or the time at which said signals are generated and the influence one of said signals has upon another of said signals.

3. The method of claim 1 wherein said signals are fluorescent.

4. The method of claim 3 wherein said signals have the same wavelength.

5. The method of claim 1 wherein said common capture reagent comprises at least one binding member attached to a solid phase.

6. The method of claim 1 wherein at least one indicator reagent comprises a steady state detectable moiety and at least one indicator reagent comprises a time dependent detectable moiety.

7. A method for determining the presence or amount of at least two nucleic acid sequences that may be present in a test sample, wherein at least one of said at least two nucleic acid sequences comprises one of an enzymatic label or a fluorophore, said method comprising the steps of:
(a) contacting said test sample with a common capture reagent for a time and under conditions sufficient to form capture reagent/analyte complexes wherein said common capture reagent includes one or more specific binding members that bind at least two analytes that may be present in said test sample;
(b) contacting said capture reagent/analyte complexes with at least one indicator reagent, comprising the other one of an enzymatic label or a fluorophore, for a time and under conditions sufficient to form capture reagent/analyte/indicator reagent complexes ; and
(c) detecting at least two measurable signals as a measure of the presence or amount of said sequences in said test sample;
wherein said signals are detected simultaneously, or simultaneously and sequentially, and
wherein said nucleic acid sequences comprise a capture region and a reporter region.

8. The method of claim 7 wherein at least one reporter region comprises a steady state detectable moiety.

9. The method of claim 8 wherein at least one reporter region comprises a binding member and said indicator reagent comprises an antibody attached to a time dependent detectable moiety.

## Patentansprüche

1. Ein Verfahren zur Bestimmung der Anwesenheit oder Menge von mindestens zwei Analyten, die in einer Testprobe anwesend sein können, wobei das Verfahren folgende Schritte umfaßt:
(a) In-Kontakt-bringen der Testprobe mit einem herkömmlichen Einfangreagenz für einen Zeitraum und unter Bedingungen, die ausreichend sind, um Einfangreagenz/Analyt-Komplexe zu bilden, worin das herkömmliche Einfangreagenz ein oder mehrere spezifische Bindungsglieder besitzt, die mindestens zwei Analyten binden, die in der Testprobe anwesend sein können;
(b) In-Kontakt-bringen der Einfangreagenz/Analyt-Komplexe mit mindestens zwei Indikatorreagenzien für einen Zeitraum und unter Bedingungen, die ausreichend sind, um Einfangreagenz/Analyt/Indikatorreagenz-Komplexe zu bilden, worin mindestens eines der mindestens zwei Indikatorreagenzien eine enzymatische Markierung umfaßt und mindestens eines der mindestens zwei Indikatorreagenzien ein Fluorophor umfaßt; und
(c) Detektieren von mindestens zwei meßbaren Signalen als ein Maß für die Anwesenheit oder Menge der Analyten in der Testprobe.

2. Das Verfahren gemäß Anspruch 1, worin sich die Signale voneinander unterscheiden basierend auf dem Einfluß, den eines der Signale auf ein anderes der Signale hat; oder basierend auf dem Zeitpunkt, zu welchem die Signale erzeugt werden und dem Einfluß, den eines der Signale auf ein anderes der Signale hat.

3. Das Verfahren gemäß Anspruch 1, worin die Signale fluoreszierend sind.

4. Das Verfahren gemäß Anspruch 3, worin die Signale die selbe Wellenlänge haben.

5. Das Verfahren gemäß Anspruch 1, worin das herkömmliche Einfangreagenz mindestens ein Bindeglied umfaßt, das an eine feste Phase angeheftet ist.

6. Das Verfahren gemäß Anspruch 1, worin mindestens ein Indikatorreagenz einen stationären detektierbaren Anteil umfaßt und mindestens ein Indikatorreagenz einen zeitabhängigen detektierbaren Anteil umfaßt.

7. Ein Verfahren zur Bestimmung der Anwesenheit oder Menge von mindestens zwei Nukleinsäuresequenzen, die in einer Testprobe anwesend sein können, worin mindestens eine der mindestens zwei Nukleinsäuresequenzen eines von einer enzymatischen Markierung oder einem Fluorophor umfaßt, wobei das Verfahren folgende Schritte umfaßt:
(a) In-Kontakt-bringen der Testprobe mit einem herkömmlichen Einfangreagenz für einen Zeitraum und unter Bedingungen, die ausreichend sind, um Einfangreagenz/Analyt-Komplexe zu bilden, worin das herkömmliche Einfangreagenz ein oder mehrere spezifische Bindungsglieder besitzt, die mindestens zwei Analyten binden, die in der Testprobe anwesend sein können;
(b) In-Kontakt-bringen der Einfangreagenz/Analyt-Komplexe mit mindestens einem Indikatorreagenz, das das andere von einer enzymatischen Markierung oder einem Fluorophor umfaßt, für einen Zeitraum und unter Bedingungen, die ausreichend sind, um Einfangreagenz/Analyt/Indikatorreagenz-Komplexe zu bilden; und
(c) Detektieren von mindestens zwei meßbaren Signalen als ein Maß für die Anwesenheit oder Menge der Sequenzen in der Testprobe;
wobei die Signale gleichzeitig detektiert werden, oder gleichzeitig und nacheinander, und
wobei die Nukleinsäuresequenzen eine Einfangregion und eine Reporterregion umfassen.

8. Das Verfahren gemäß Anspruch 7, wobei mindestens eine Reporterregion einen stationären detektierbaren Anteil umfaßt.

9. Das Verfahren gemäß Anspruch 8, worin mindestens eine Reporterregion ein Bindeglied umfaßt und das Indikatorreagenz einen Antikörper umfaßt, der an einen zeitabhängigen detektierbaren Anteil angeheftet ist.

## Revendications

1. Procédé pour déterminer la présence ou la quantité d'au moins deux analytes qui peuvent être présents dans un échantillon test, ledit procédé comprenant les étapes :
(a) de mise en contact dudit échantillon test avec un réactif de capture commun pendant un laps de temps et sous des conditions suffisantes pour former des complexes réactif de capture/analyte, dans lesquels ledit réactif de capture commun comprend un ou plusieurs membres spécifiques de liaison qui lient au moins deux analytes qui peuvent être présents dans ledit échantillon test ;
(b) de mise en contact desdits complexes réactif de capture/analyte avec au moins deux réactifs indicateurs pendant un laps de temps et sous des conditions suffisantes pour former des composés réactif de capture/analyte/réactif indicateur, dans lesquels au moins un desdits au moins deux réactifs indicateurs comprend un marqueur enzymatique et au moins un desdits au moins deux réactifs indicateurs comprend un fluorophore ; et
(c) de détection d'au moins deux signaux mesurables comme mesure de la présence ou d'une quantité desdits analytes dans ledit échantillon test.

2. Procédé selon la revendication 1, dans lequel lesdits signaux sont différenciés sur base de l'influence qu'un desdits signaux a sur l'autre desdits signaux ; ou du temps auquel lesdits signaux sont générés et l'influence qu'un desdits signaux a sur un autre desdits signaux.

3. Procédé selon la revendication 1, dans lequel lesdits signaux sont fluorescents.

4. Procédé selon la revendication 3, dans lequel lesdits signaux ont la même longueur d'onde.

5. Procédé selon la revendication 1, dans lequel ledit réactif de capture commun comprend au moins un membre de liaison attaché à une phase solide.

6. Procédé selon la revendication 1, dans lequel au moins un réactif indicateur comprend une moitié détectable à l'état stable et au moins un réactif indicateur comprend une moitié détectable en fonction du temps.

7. Procédé pour déterminer la présence ou la quantité d'au moins deux séquences d'acides nucléiques qui peuvent être présentes dans un échantillon test, dans lequel au moins une desdites au moins deux séquences d'acides nucléiques comprend un parmi un marqueur enzymatique ou un fluorophore, ledit procédé comprenant les étapes :
(a) de mise en contact dudit échantillon test avec un réactif de capture commun pendant un laps de temps et sous des conditions suffisantes pour former des complexes réactif de capture/analyte, dans lesquels ledit réactif de capture commun comprend un ou plusieurs membres spécifiques de liaison qui lient au moins deux analytes qui peuvent être présents dans ledit échantillon test ;
(b) de mise en contact desdits complexes réactif de capture/analyte avec au moins un réactif indicateur comprenant l'autre parmi un marqueur enzymatique ou un fluorophore, pendant un laps de temps et sous des conditions suffisantes pour former des composés réactif de capture/analyte/réactif indicateur ; et
(c) de détection d'au moins deux signaux mesurables comme mesure de la présence ou d'une quantité desdites séquences dans ledit échantillon test ;
dans lequel lesdits signaux sont détectés simultanément, ou simultanément et séquentiellement et
dans lequel lesdites séquences d'acides nucléiques comprennent une région de capture et une région de reconnaissance.

8. Procédé selon la revendication 7, dans lequel au moins une région de reconnaissance comprend une moitié détectable à l'état stable.

9. Procédé selon la revendication 8, dans lequel au moins une région de reconnaissance comprend un membre de liaison et ledit réactif indicateur comprend un anticorps attaché à une moitié détectable en fonction du temps.
